(19) Europäisches Patentamt
European Patent Office
Office européen des brevets

(11) EP 4 592 332 A1

(12) **EUROPEAN PATENT APPLICATION**
published in accordance with Art. 153(4) EPC

(43) Date of publication:
**30.07.2025 Bulletin 2025/31**

(21) Application number: **23907846.2**

(22) Date of filing: **22.12.2023**

(51) International Patent Classification (IPC):
***C08G 63/60*** (2006.01) ***C08G 63/82*** (2006.01)
***C08L 67/02*** (2006.01) ***C08K 5/05*** (2006.01)

(52) Cooperative Patent Classification (CPC):
**C08G 63/60; C08G 63/82; C08K 5/05; C08L 67/02**

(86) International application number:
**PCT/KR2023/021358**

(87) International publication number:
**WO 2024/136563 (27.06.2024 Gazette 2024/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.12.2022 KR 20220183172**

(71) Applicant: **LG CHEM, LTD.**
**Seoul 07336 (KR)**

(72) Inventors:
- **LEE, Yeonju**
  **Daejeon 34122 (KR)**
- **KIM, Si Min**
  **Daejeon 34122 (KR)**
- **JEONG, Yongbok**
  **Daejeon 34122 (KR)**
- **KANG, Donggyun**
  **Daejeon 34122 (KR)**
- **KIM, Chul Woong**
  **Daejeon 34122 (KR)**

(74) Representative: **Plasseraud IP**
**104 Rue de Richelieu**
**CS92104**
**75080 Paris Cedex 02 (FR)**

(54) **METHOD FOR PREPARING ALKYL POLY(3-HYDROXYPROPIONATE), ALKYL POLY(3-HYDROXYPROPIONATE), AND COMPOSITION COMPRISING SAME**

(57) The present disclosure provides a method for preparing an alkyl poly(3-hydroxypropionate) comprising: subjecting an alkyl-3-hydroxypropionate to condensation polymerization to prepare an alkyl poly(3-hydroxypropionate), wherein the alkyl-3-hydroxypropionate has 2 to 20 carbon atoms in the alkyl, an alkyl poly(3-hydroxypropionate) and a composition comprising the same.



Processed by Luminess, 75001 PARIS (FR)

## Description

## FIELD OF THE INVENTION

CROSS-REFERENCE TO RELATED APPLICATION(S)

This application claims priority to and the benefit of Korean Patent Application No. 10-2022-0183172, filed on December 23, 2023, with the Korean Intellectual Property Office, the disclosure of which is incorporated herein by reference in its entirety.

The present disclosure relates to a method for preparation of alkyl poly(3-hydroxypropionate), an alkyl poly(3-hydroxypropionate) and a composition comprising the same.

## BACKGROUND OF THE INVENTION

Poly(3-hydroxypropionic acid), which is a biodegradable polymer, not only has the property of being not easily broken but also has excellent mechanical properties, and therefore, is attracting attention as an environmentally friendly material.

Poly(3-hydroxypropionic acid) is prepared by the condensation polymerization of 3-hydroxypropionic acid (3-HP), wherein a method of preparing the 3-hydroxypropionic acid by microbial fermentation has been attracting attention as an environmentally friendly bioprocess.

However, when the 3-hydroxypropionic acid is prepared by microbial fermentation, by-products other than 3-hydroxypropionic acid are also produced in the process of fermenting microorganisms, and thus, various processes are required to separate and purify 3-hydroxypropionic acid from a fermented liquid. In particular, 3-hydroxypropionic acid exhibits high hydrophilicity and has high solubility and reactivity in water, and thus, separation and purification are not easy.

As an example for recovering 3-hydroxypropionic acid, 3-hydroxypropionic acid can be converted into alkyl-3-hydroxypropionate and purified, but additional steps such as removing the alkyl group therefrom is needed.

## SUMMARY OF THE INVENTION

## TECHNICAL PROBLEM

It is an object of the present disclosure to provide a method for preparing an alkyl poly(3-hydroxypropionate) by the condensation polymerization of alkyl-3-hydroxypropionate, an alkyl poly(3-hydroxypropionate) prepared thereby, and a composition comprising the same.

## TECHNICAL SOLUTION

According to an embodiment of the present disclosure, there is provided a method for preparing an alkyl poly(3-hydroxypropionate) comprising: subjecting an alkyl-3-hydroxypropionate to condensation polymerization to prepare an alkyl poly(3-hydroxypropionate) represented by the following Chemical Formula 1, wherein the alkyl-3-hydroxypropionate has 2 to 20 carbon atoms in the alkyl.

According to another embodiment of the present disclosure, there is provided an alkyl poly(3-hydroxypropionate) represented by the following Chemical Formula 1.

According to yet another embodiment of the present disclosure, there is provided an alkyl poly(3-hydroxypropionate) composition comprising: an alkyl poly(3-hydroxypropionate) represented by the following Chemical Formula 1, and an alcohol having 2 to 20 carbon atoms.

Now, a method for preparing an alkyl poly(3-hydroxypropionate), an alkyl poly(3-hydroxypropionate) and a composition comprising the same according to specific embodiments of the present disclosure will be described in more detail.

Further, the steps constituting the preparation method described herein are not construed as being limited to the order in which one step and the other steps constituting one preparation method are described herein, unless explicitly stated as being sequential or continuous order or otherwise specified. Therefore, the order of the constituent steps of the preparation method can be changed within a range that can be easily understood by those skilled in the art, and in this case, changes apparent to those skilled in the art accompanying therewith are included in the scope of the invention.

Unless particularly mentioned herein, the term "including" or "comprising" refers to including some element (or component) without any limitation, and should not be construed as excluding addition of other elements (or components).

Further, unless otherwise stated herein, the weight average molecular weight and number average molecular weight of alkyl poly(3-hydroxypropionate), etc. can be measured using a gel permeation chromatography(GPC).

Specifically, the polymer or copolymer is dissolved in chloroform to a concentration of 1 mg/ml, then 100 $\mu\ell$ of the solution is injected into GPC, and GPC analysis is carried out at 40°C. At this time, chloroform is used as the mobile phase for GPC, the flow rate is 1.0 mL/min, and the column used is two Agilent Mixed-B units connected in series. RI Detector is used as a detector. The values of Mw can be obtained using a calibration curve formed using a polystyrene standard sample. Twelve kinds of the polystyrene standard samples are used with the weight average molecular weight of 162 g/mol, 580 g/mol, 1,180 g/mol, 4,870 g/mol, 9,310 g/mol, 17,120 g/mol, 75,050 g/mol, 200,500 g/mol, 448,500 g/mol, 10,690,000 g/mol, 3,022,000 g/mol, and 6,545,000 g/mol.

According to an embodiment of the present disclosure, there is provided a method for preparing an alkyl poly(3-hydroxypropionate) comprising: subjecting an alkyl-3-hydroxypropionate to condensation polymerization to prepare an alkyl poly(3-hydroxypropionate) represented by the following Chemical Formula 1, wherein the alkyl-3-hydroxypropionate has 2 to 20 carbon atoms in the alkyl.

[Chemical Formula 1]

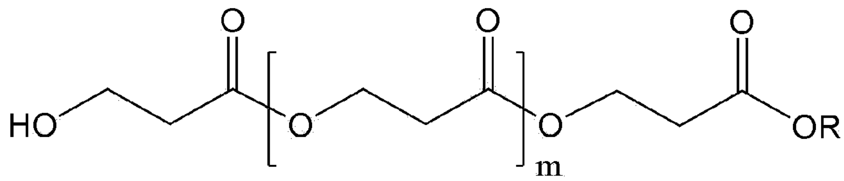

wherein in Chemical Formula 1,

R is a linear or branched alkyl group represented by $C_nH_{2n+1}$ (where n is an integer of 2 to 20), and
m is an integer of 10 or more.

The present inventors have found that, when the alkyl poly(3-hydroxypropionate) is prepared by the condensation polymerization of alkyl-3-hydroxypropionate, an alcohol having a lower boiling point than water is produced as a condensation polymerization by-product, thereby being able to effectively remove by-products, and the final product of alkyl poly(3-hydroxypropionate) has a structure in which the carboxyl group(-COOH) is capped with an alkyl group, so that the acid value of the polymer is low, the content of vinyl groups at the end groups is low, and the yield finally recovered is also excellent, and completed the present disclosure.

In the method for preparing an alkyl poly(3-hydroxypropionate) according to one embodiment, the alkyl-3-hydroxypropionate may be subjected to condensation polymerization to prepare the alkyl poly(3-hydroxypropionate) represented by Chemical Formula 1.

When the alkyl-3-hydroxypropionate is subjected to condensation polymerization to prepare an alkyl poly(3-hydroxypropionate), alcohol is generated as a condensation polymerization by-product. However, since this alcohol has a lower boiling point than water, it can be efficiently removed even at low temperatures, the content of by-products finally generated is small, and the water removal step that is conventionally involved in the production of poly(3-hydroxypropionate) can be omitted.

Further, the alkyl poly(3-hydroxypropionate) has a structure in which the carboxyl group (carboxyl acid group) at the end group is substituted with an alkyl ester, and as a result, the carboxyl end-group is capped, so that the acid value may be lowered. As the acid value of the alkyl poly(3-hydroxypropionate) is lowered, the storage stability of the polymer can be improved and the possibility of side reactions can be reduced. Further, an additive for lowering the acid value is conventionally added in order to control the acid value of the polymer, but the preparation method according to one embodiment can lower the acid value without adding an additional additive, thereby reducing the occurrence of by-products.

Meanwhile, the acid value of the alkyl poly(3-hydroxypropionate) may show different tendencies according to the number average molecular weight. For example, when the number average molecular weight of the alkyl poly(3-hydroxypropionate) is more than 3,000 g/mol, the acid value may be higher as the number average molecular weight is lower.

However, the alkyl poly(3-hydroxypropionate) prepared by the preparation method according to one embodiment may show a low acid value of 300.0 meq/Kg or less even if the number average molecular weight exceeds 3,000 g/mol. For example, the alkyl poly(3-hydroxypropionate) having a number average molecular weight of more than 3,000 g/mol and 8,000 g/mol or less may have an acid value of 300.0 meq/Kg or less, 1.0 meq/Kg or more and 290.0 meq/Kg or less, and 10.0 meq/Kg or more, 20.0 meq/Kg or more, 30.0 meq/Kg or more, 50.0 meq/Kg or more, and 280.0 meq/Kg or less, 270.0 meq/Kg or less, 250.0 meq/Kg or less, 230.0 meq/Kg or less, or 210.0 meq/Kg or less. Further, the alkyl poly(3-hydroxypropionate) having a number average molecular weight of more than 8,000 g/mol and 30,000 g/mol or less may

have an acid value of 150.0 meq/Kg or less, or 1.0 meq/Kg or more and 140.0 meq/Kg or less, and 10.0 meq/Kg or more, 20.0 meq/Kg or more, and 145.0 meq/Kg or less, 140.0 meq/Kg or less, 135.0 meq/Kg or less, or 130.0 meq/Kg or less.

Meanwhile, the alkyl poly(3-hydroxypropionate) having a number average molecular weight of 300 g/mol or more and 3,000 g/mol or less may have an acid value of 1.0 meq/Kg or more and 333.0 meq/Kg or less, and 2.0 meq/Kg or more, 3.0 meq/Kg or more, or 300.0 meq/Kg or less, 200.0 meq/Kg or less, 100.0 meq/Kg or less, 70.0 meq/Kg or less, 50.0 meq/Kg or less, 30.0 meq/Kg or less, or 20.0 meq/Kg or less.

Further, the alkyl poly(3-hydroxypropionate) is prepared by the condensation polymerization of the alkyl-3-hydroxypropionate, so that the content of vinyl groups at the end of the alkyl poly(3-hydroxypropionate) may be 40 mol% or less. For example, the content of vinyl groups at the end of the alkyl poly(3-hydroxypropionate) may be 30 mol% or less, 20 mol% or less, 15 mol% or less, 10 mol% or less, or 5 mol% or less, or 0.01 mol% or more, 0.02 mol% or more, 0.03 mol% or more, 0.04 mol% or more, or 0.05 mol% or more.

The alkyl poly(3-hydroxypropionate) may have a hydroxy group, a carboxy group, a vinyl group, etc. as an end group, but since the alkyl poly(3-hydroxypropionate) is prepared by the condensation polymerization of the alkyl 3-hydroxypropionate, there is a low possibility that a vinyl group will exist in the end group. The vinyl group is generated due to a side reaction that occurs during the polymerization of 3-hydroxypropionic acid into poly(3-hydroxypropionic acid), wherein the vinyl group acts as a factor that breaks the equivalent ratio in condensation polymerization, where the equivalent ratio of each functional group (hydroxyl group, carboxyl group) is important, thereby inhibiting the reaction rate and making it difficult to obtain a polymer with a high molecular weight. Furthermore, if a vinyl group exists in the alkyl poly(3-hydroxypropionate), there is a problem that chain extension of the alkyl poly(3-hydroxypropionate) through further modification does not favorably occur. Therefore, since the alkyl poly(3-hydroxypropionate) has a small number of vinyl groups in the end groups, it has the advantage that the above-mentioned drawbacks are fundamentally reduced.

The content of the end group of the poly(3-hydroxypropionic acid) can be determined by calculating the ratio of vinyl groups to the total end groups through $^1H$-NMR measurement using a Buker 500MHz NMR model equipment. For example, the polymer can be dissolved in d-$CDCl_3$ at a concentration of 8 mg/ml and measured, and calculated according to the following Mathematical Equation 1

[Mathematical Equation 1]

$$vinyl\ group(\%) = \frac{2a}{(2a+b)} \times 100$$

in Mathematical Equation 1,

a is the area value of $^1H$ in C=C of 6.3 ppm,
b is the area value of $^2H$ in HO-$CH_2$- of 3.8 ppm.

The area value of $^1H$ in C=C is the area value of hydrogen on the side closer to the carbonyl group among hydrogens(H) of the double bond of the vinyl group, and the area value of $^2H$ in HO-$CH_2$- may be the area value of two hydrogens substituted on the carbon connected to the end of the hydroxyl group.

The alkyl-3-hydroxypropionate may have 2 to 20, 2 to 10, 2 to 8, or 2 to 6 carbon atoms in the alkyl, and an example thereof may be ethyl, n-propyl, isopropyl, n-butyl, or tert-butyl.

Further, the alkyl poly(3-hydroxypropionate) may be represented by the following Chemical Formula 1.

[Chemical Formula 1]

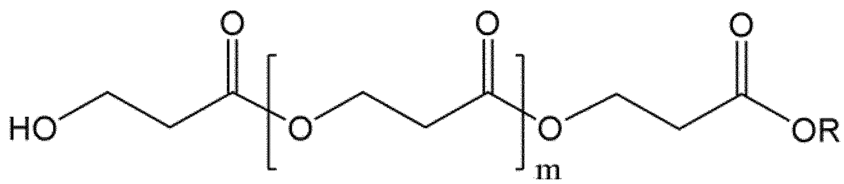

The R is an alkyl group capping a carboxyl group of poly(3-hydroxypropionate), and may be derived from the alkyl of alkyl-3-hydroxypropionate. For example, the R may be a linear or branched alkyl group represented by $C_nH_{2n+1}$ (where n is an integer of 2 to 20), a linear or branched alkyl group represented by $C_nH_{2n+1}$ (where n is an integer of 2 to 10), or a linear or branched alkyl group represented by $C_nH_{2n+1}$ (where n is an integer of 2 to 6), and more specifically, it may be an

ethyl group, an n-propyl group, an isopropyl group, an n-butyl group, or a tert-butyl group.

The m represents the number of repeating structures, and for example, m may be an integer of 10 or more, or an integer of 10 to 600.

The alkyl poly(3-hydroxypropionate) may have a weight average molecular weight of 500 g/mol or more, 600 g/mol or more, 1,000 g/mol or more, 2,000 g/mol or more, 3,000 g/mol or more, or 5,000 g/mol or more, and 100,000 g/mol or less, 80,000 g/mol or less, 70,000 g/mol or less, 60,000 g/mol or less, 50,000 g/mol or less, 25,000 g/mol or less, or 20,000 g/mol or less. If the weight average molecular weight of the alkyl poly(3-hydroxypropionate) is too low, it is low in crystallinity and does not exist in a solid state, and may be easily broken and be reduced in strength, and if the weight average molecular weight is too high, it may have high viscosity and be difficult to be processed.

The alkyl poly(3-hydroxypropionate) may have a number average molecular weight of 300 g/mol or more, 400 g/mol or more, 500 g/mol or more, 1,000 g/mol or more, 2,000 g/mol or more, 3,000 g/mol or more, or 5,000 g/mol or more, and 30,000 g/mol or less, 25,000 g/mol or less, 20,000 g/mol or less, 15,000 g/mol or less, 10,000 g/mol or less, 9,000 g/mol or less, or 8,000 g/mol or less. If the number average molecular weight of the alkyl poly(3-hydroxypropionate) is too low, it is low in crystallinity and does not exist in a solid state, and may be easily broken and be reduced in strength, and if the number average molecular weight is too high, it may have high viscosity and be difficult to be processed.

Further, the alkyl poly(3-hydroxypropionate) may have a number average molecular weight and a molecular weight distribution (Mw/Mn) of 1.0 to 35.0. More preferably, the molecular weight distribution of the poly(3-hydroxypropionic acid) according to the present disclosure may be 1.3 or more, 1.4 or more, 1.7 or more, 1.8 or more, or 2.0 or more, and 35.0 or less, 30.0 or less, 25.0 or less, 20.0 or less, 11.0 or less, 5.0 or less, 3.0 or less, or 2.5 or less.

The alkyl poly(3-hydroxypropionate) prepared by the preparation method according to one embodiment can be prepared by subjecting alkyl-3-hydroxypropionate to condensation polymerization reaction. Specifically, the condensation polymerization of the alkyl-3-hydroxypropionate can be carried out at a temperature of 50°C or more and 250°C or less. Further, the condensation polymerization may be carried out for 6 hours or more and 30 hours or less.

For example, the condensation polymerization may be carried out at a temperature of 50°C or more, or 70°C or more, or 90°C or more, or 110°C or more, and at a temperature of 250°C or less, or 230°C or less, or 200°C or less, for a time period of 6 hours or more, or 8 hours or more, or 10 hours or more, and 30 hours or less, or 28 hours or less, or 26 hours or less. When the polymerization is carried out under the above conditions, the alkyl poly(3-hydroxypropionate) having the physical properties and weight average molecular weight in the optimal range to be realized in the present disclosure can be prepared in an excellent yield.

The condensation polymerization may be carried out in the presence of at least one catalyst selected from the group consisting of sulfonic acid-based catalyst, metal oxide, metal chloride, and metal alkoxide catalysts.

The sulfonic acid catalyst may include, not is limited thereto, for example, benzenesulfonic acid, n-butylbenzenesulfonic acid, n-octylbenzenesulfonic acid, n-dodecylbenzenesulfonic acid, pentadecylbenzenesulfonic acid, 2,5-dimethylbenzenesulfonic acid, 2,5-dibutylbenzenesulfonic acid, o-aminobenzenesulfonic acid, m-aminobenzenesulfonic acid, p-aminobenzenesulfonic acid, 3-amino-4-hydroxybenzenesulfonic acid, 5-amino-2-methylbenzenesulfonic acid, 3,5-diamino-2,4,6-trimethylbenzenesulfonic acid, 2,4-dinitrobenzenesulfonic acid, p-chlorobenzenesulfonic acid, 2,5-dichlorobenzenesulfonic acid, hydroxynitrobenzenesulfonic acid, aminotoluenesulfonic acid, p-phenolsulfonic acid, aminophenolsulfonic acid, cumenesulfonic acid, xylenesulfonic acid, o-cresolsulfonic acid, m-cresolsulfonic acid, p-cresolsulfonic acid, p-toluenesulfonic acid (p-TSA), methanesulfonic acid (m-SA), trifluoromethanesulfonic acid, nonafluorobutane-1-sulfonic acid, 2-naphthalenesulfonic acid, p-xylene-4-sulfonic acid, 2-toluenesulfonic acid, 3-toluenesulfonic acid, 2-ethylbenzenesulfonic acid, 3-ethylbenzenesulfonic acid, 4-ethylbenzenesulfonic acid, taurine, cyclopentanesulfonic acid, cyclohexanesulfonic acid, sulfuric acid, camphorsulfonic acid, and the like.

The metal oxide may include, but is not limited to, germanium dioxide, zinc oxide, tin oxide, antimony trioxide, iron trioxide, aluminum trioxide, silicon dioxide, titanium dioxide, and the like.

The metal alkoxide may include, but is not limited to, for example, titanium butoxide, titanium isopropoxide, aluminum isopropoxide, yttrium isopropoxide, germanium ethoxide, silicon ethoxide, tin octoate, and the like.

The metal chloride may include, but is not limited to, potassium chloride, calcium chloride, nickel chloride, cobalt chloride, magnesium chloride, manganese chloride, iron chloride, barium chloride, zinc chloride, aluminum chloride, tin chloride, and the like.

The catalyst may be used in an amount of 0.001 mol% or more and 10 mol% or less based on the alkyl-3-hydroxypropionate. For example, the catalyst may be used in an amount of 0.010 mol% or more, or 0.050 mol% or more, and 0.10 mol% or more, or 0.20 mol% or more, or 10 mol% or less, 5 mol% or less, 3 mol% or less, or 1 mol% or less based on the alkyl-3-hydroxypropionate. If the catalyst is used in an excessively small amount, the polymerization activity may not be sufficient, and if the catalyst is used in an excessively large amount, the amount of residual catalyst may become larger, which may result in decomposition of the polymer or a decrease in molecular weight due to depolymerization such as transesterification reaction.

Further, the preparation method according to one embodiment may include a (step 1) melt polymerizing an alkyl-3-hydroxypropionate to prepare an alkyl-3-hydroxypropionic acid oligomer; and (step 2) further polymerizing the

alkyl-3-hydroxypropionic acid oligomer to prepare an alkyl poly(3-hydroxypropionate).

The melt polymerization means that the reactant alkyl-3-hydroxypropionate and the product alkyl-3-hydroxypropionic acid oligomer maintain a liquid state. For this purpose, in the present disclosure, the reaction temperature of the step 1 is adjusted to a temperature of 40°C to 120°C. Although not theoretically limited, the formation of cyclic oligomers is suppressed in the process for polymerizing alkyl-3-hydroxypropionate under the melt polymerization conditions. Preferably, the reaction temperature of the step 1 may be 40°C or more, 45°C or more, 50°C or more, 55°C or more, 60°C or more, 65°C or more, 70°C or more, 75°C or more, or 80°C or more, and 135°C or less, 130°C or less, 125°C or less, 120°C or less, 115°C or less, 110°C or less, 105°C or less, 100°C or less, or 95°C or less.

The step 1 can be carried out under a pressure of 5 mbar to 200 mbar. When the reaction of the step 1 is carried out under such low pressures, the polymerization of alkyl-3-hydroxypropionate can be promoted. More preferably, the step 1 can be carried out at 6 mbar or more, or 7 mbar or more, and 190 mbar or less, 180 mbar or less, 170 mbar or less, 160 mbar or less, 150 mbar or less, 140 mbar or less, 130 mbar or less, 120 mbar or less, 110 mbar or less, 100 mbar or less, 90 mbar or less, 80 mbar or less, 70 mbar or less, 60 mbar or less, 50 mbar or less, 40 mbar or less, 30 mbar or less, 20 mbar or less, or 10 mbar or less.

The reaction time of the step 1 can be appropriately determined in consideration of the molecular weight, yield, etc. of the alkyl-3-hydroxypropionic acid oligomer produced, and preferably, the reaction is carried out for 1 to 5 hours. Within the above reaction time, the molecular weight of the alkyl-3-hydroxypropionic acid oligomer can be increased to an appropriate level, and the production yield can also be improved.

Further, the catalyst can be added in a predetermined amount during the reaction of the step 1. The catalyst has the effect of promoting the polymerization of the alkyl-3-hydroxypropionate and, at the same time, suppressing the formation of a cyclic oligomer during the polymerization process of the alkyl-3-hydroxypropionate.

Meanwhile, for the melt polymerization of the step 1, if necessary, a step of drying the alkyl-3-hydroxypropionate may be carried out before carrying out the step 1. Through the drying, moisture present in the alkyl-3-hydroxypropionate can be removed. At this time, the drying temperature is preferably 40 to 95°C, and the drying pressure is preferably 10 mbar to normal pressure, and the drying time is preferably 1 to 10 hours.

In the step 2, the alkyl-3-hydroxypropionic acid oligomer can be further polymerized to prepare an alkyl poly(3-hydroxypropionate).

Since the reactant is an oligomer unlike the step 1, the step 2 can be carried out with raising the polymerization temperature and further reducing the pressure as compared with the step 1. Preferably, the polymerization temperature of the step 2 may be 50°C or more, or 70°C or more, or 90°C or more and 250°C or less, or 230°C or less, 200°C or less, 180°C or less, or 160°C or less.

The pressure of the step 2 is 1 mbar or less, and more preferably, the pressure of the step 2 is 0.9 mbar or less, 0.8 mbar or less, 0.7 mbar or less, 0.6 mbar or less, 0.5 mbar or less, 0.4 mbar or less, 0.3 mbar or less, 0.25 mbar or less, 0.20 mbar or less, 0.19 mbar or less, or 0.18 mbar or less, and 0.01 mbar or more, 0.02 mbar or more, 0.03 mbar or more, 0.04 mbar or more, 0.05 mbar or more, 0.06 mbar or more, 0.07 mbar or more, 0.08 mbar or more, 0.09 mbar or more, or 0.01 mbar or more.

The reaction time of the step 2 can be appropriately determined in consideration of the molecular weight, yield, etc. of the alkyl poly(3-hydroxypropionate) produced, and the reaction is preferably carried out for a time period of 6 hours or more, or 8 hours or more, or 10 hours or more, and 30 hours or less, or 28 hours or less, or 26 hours or less. Within the above reaction time, the molecular weight of the alkyl poly(3-hydroxypropionate) can be increased to an appropriate level, and the production yield can also be improved.

Meanwhile, since the step 2 is carried out subsequent to the step 1, the catalyst added in the step 1 also participates in the reaction even in the step 2. Therefore, the catalyst described in the previous step 1 can also be applied in the step 2.

According to another embodiment of the disclosure, the alkyl poly(3-hydroxypropionate) represented by the following Chemical Formula 1 is provided.

[Chemical Formula 1]

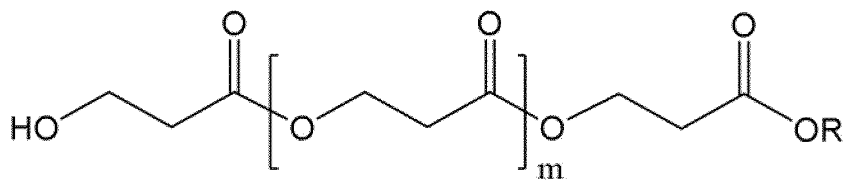

wherein in Chemical Formula 1,

R is a linear or branched alkyl group represented by $C_nH_{2n+1}$ (where n is an integer of 2 to 20), and
m is an integer of 10 or more.

The alkyl poly(3-hydroxypropionate) may be prepared by the method for preparing the alkyl poly(3-hydroxypropionate) according to one embodiment.

Further, the acid value, the content of vinyl groups in the end groups, the weight average molecular weight, and the number average molecular weight of the alkyl poly(3-hydroxypropionate) are as described above.

Further, according to yet another embodiment of the disclosure, there is provided an alkyl poly(3-hydroxypropionate) composition comprising the alkyl poly(3-hydroxypropionate) represented by Chemical Formula 1, and an alcohol having 2 to 20 carbon atoms.

The composition may be prepared by the method for preparing an alkyl poly(3-hydroxypropionate) according to one embodiment.

The preparation method prepares a poly(3-hydroxypropionate) through condensation polymerization of an alkyl-3-hydroxypropionate, and thus, an alcohol having 2 to 20 carbon atoms can be produced as a by-product. The alcohol has a lower boiling point than water, and can be efficiently removed even at a low temperature, so that the content of the by-product finally generated is small, and the water removal process that is conventionally involved in the preparation of the poly(3-hydroxypropionate) can be omitted.

The alcohol can have 2 to 20 carbon atoms, 2 to 10 carbon atoms, 2 to 8 carbon atoms, or 2 to 6 carbon atoms, and, for example, the alcohol can be ethanol, n-propanol, isopropanol, n-butanol, or tert-butanol.

## ADVANTAGEOUS EFFECTS

The present disclosure provides a method for preparing an alkyl poly(3-hydroxypropionate), an alkyl poly(3-hydroxypropionate) and a composition comprising the same, wherein the method prepares an alkyl poly(3-hydroxypropionate) by the condensation polymerization of an alkyl-3-hydroxypropionate and efficiently removes by-products such as alcohol, and the acid value of the finally prepared polymer is low, and the formation of vinyl groups due to side reactions is small.

## DETAILED DESCRIPTION OF THE EMBODIMENTS

Hereinafter, embodiments of the present disclosure will be explained in detail with reference to examples. However, the following examples are for illustrative purposes only, and the detailed description of the present disclosure is not limited by these examples.

### Experimental Example 1

15 g of ethyl-3-hydroxypropionate from which moisture was removed was placed in a reactor, and 0.4 mol% of octyltriamine as a catalyst was added based on ethyl-3-hydroxypropionate. The temperature and pressure inside the reactor were maintained at 90°C and 10 torr, respectively, and the reaction was carried out for 2 hours to prepare an ethyl-3-hydroxypropionate oligomer. Then, the temperature and pressure inside the reactor were adjusted to 90°C and 0.2 torr, respectively, and the reaction was carried out for 24 hours to prepare an ethyl poly(3-hydroxypropionate).

### Experimental Example 2

Ethyl poly(3-hydroxypropionate) was prepared in the same manner as in Experimental Example 1, except that p-TSA (p-toluenesulfonic acid) was used instead of octyltriamine.

### Experimental Example 3

Ethyl poly(3-hydroxypropionate) was prepared in the same manner as in Experimental Example 1, except that tin chloride($SnCl_2$) was used instead of octyltriamine.

### <Evaluation>

### 1. Measurement of gas chromatography (GC)

The reaction products of Experimental Examples 1 to 3 were measured by gas chromatography(GC), and the content of by-products produced in addition to the final ethyl poly(3-hydroxypropionate), such as ethanol, ethyl-3-hydroxypropionate, dimer, and other by-products, was measured. The results are shown in Table 1 below.

[Table 1]

| Unit(wt.%) | Ethanol | Ethyl-3-hydroxypropionate | Dimer | Other |
|---|---|---|---|---|
| Experimental Example 1 | 0.8 | 99.2 | 0 | 0 |
| Experimental Example 2 | 27.0 | 47.8 | 25.2 | 0 |
| Experimental Example 3 | 3.6 | 64.5 | 31.2 | 0.7 |

According to Table 1, it was confirmed that in Experimental Examples 2 and 3, the starting material ethyl-3-hydroxypropionate was measured to be less than 64.5 wt.%, while in Experimental Example 1, ethyl-3-hydroxypropionate was measured to be 99.2 wt.%, so that the condensation polymerization reactivity was lower than that in Experimental Examples 2 and 3.

**Example 1**

15 g of ethyl-3-hydroxypropionate from which moisture was removed was placed in a reactor, and 19.7 mg of m-SA (methane sulfonic acid) as a catalyst was added at 0.2 mol% based on ethyl-3-hydroxypropionate. The temperature and pressure inside the reactor were maintained at 90°C and 10 torr, respectively, and the reaction was carried out for 2 hours to prepare an ethyl-3-hydroxypropionate oligomer. Then, the temperature and pressure inside the reactor were adjusted to 90°C and 0.2 torr, respectively, and the reaction was carried out for 24 hours to prepare an ethyl poly(3-hydroxypropionate).

**Example 2**

15 g of tert-butyl-3-hydroxypropionate from which moisture was removed was placed in a reactor, and 19.7 mg of m-SA (methane sulfonic acid) as a catalyst was added at 0.2 mol% based on tert-butyl-3-hydroxypropionate. The temperature and pressure inside the reactor were maintained at 90°C and 10 torr, respectively, and the reaction was carried out for 2 hours to prepare an ethyl-3-hydroxypropinate oligomer. Then, the temperature and pressure inside the reactor were adjusted to 90°C and 0.2 torr, respectively, and the reaction was carried out for 24 hours to prepare tert-butyl poly(3-hydroxypropionate).

**Example 3**

Tert-butyl poly(3-hydroxypropionate) was prepared in the same manner as in Example 2, except that 39.4 mg (0.4 mol%) of m-SA (methane sulfonic acid) was used instead of 19.7 mg (0.2 mol%) of m-SA (methane sulfonic acid).

**Example 4**

Tert-butyl poly(3-hydroxypropionate) was prepared in the same manner as in Example 2, except that p-TSA 63.3 mg(0.2 mol%) was used instead of 19.7 mg(0.2 mol%) of m-SA(methane sulfonic acid).

**Example 5**

Tert-butyl poly(3-hydroxypropionate) was prepared in the same manner as in Example 2, except that 126.6 mg (0.4 mol%) of p-TSA was used instead of 19.7 mg (0.2 mol%) of m-SA (methane sulfonic acid).

**Example 6**

Tert-butyl poly(3-hydroxypropionate) was prepared in the same manner as in Example 5, except that all the reaction temperatures were controlled to 120°C.

**Example 7**

Tert-butyl poly(3-hydroxypropionate) was prepared in the same manner as in Example 5, except that all the reaction temperatures were controlled to 140°C.

**Example 8**

Tert-butyl poly(3-hydroxypropionate) was prepared in the same manner as in Example 5, except that all the reaction temperatures were controlled to 160°C.

**Example 9**

Tert-butyl poly(3-hydroxypropionate) was prepared in the same manner as in Example 5, except that all the reaction temperatures were controlled to 180°C.

**Example 10**

15 g of n-butyl-3-hydroxypropionate from which moisture was removed was placed in a reactor, and 126.6 mg of p-TSA as a catalyst was added at 0.4 mol% based on n-butyl-3-hydroxypropionate. The temperature and pressure inside the reactor were maintained at 90°C and 10 torr, respectively, and the reaction was carried out for 2 hours to prepare an n-butyl-3-hydroxypropionate oligomer. Then, the temperature and pressure inside the reactor were adjusted to 90°C and 0.2 torr, respectively, and the reaction was carried out for 24 hours to prepare n-butyl poly(3-hydroxypropionate).

**Example 11**

15 g of n-butyl-3-hydroxypropionate from which moisture was removed was placed in a reactor, and titanium butoxide($Ti(BuO)_4$) as a catalyst was added at 0.4 mol% based on n-butyl-3-hydroxypropionate. The temperature and pressure inside the reactor were maintained at 90°C and 10 torr, respectively, and the reaction was carried out for 2 hours to prepare an n-butyl-3-hydroxypropionate oligomer. Then, the temperature and pressure inside the reactor were adjusted to 90°C and 0.2 torr, respectively, and the reaction was carried out for 24 hours to prepare n-butyl poly(3-hydroxypropionate).

**Example 12**

n-Butyl poly(3-hydroxypropionate) was prepared in the same manner as in Example 11, except that the reaction conditions (90°C / 0.2 torr / 24 hours) were adjusted to 120°C and 0.2 torr and the reaction was carried out for 8 hours.

**Example 13**

n-Butyl poly(3-hydroxypropionate) was prepared in the same manner as in Example 11, except that the reaction conditions (90°C / 0.2 torr / 24 hours) were adjusted to 140°C and 0.2 torr and the reaction was carried out for 8 hours.

**Example 14**

n-Butyl poly(3-hydroxypropionate) was prepared in the same manner as in Example 11, except that the reaction conditions (90°C / 0.2 torr / 24 hours) were adjusted to 160°C and 0.2 torr and the reaction was carried out for 18 hours.

**Example 15**

n-Butyl poly(3-hydroxypropionate) was prepared in the same manner as in Example 11, except that the reaction conditions (90°C / 0.2 torr / 24 hours) were adjusted to 180 °C and 0.2 torr and the reaction was carried out for 8 hours.

**Comparative Example 1**

15 g of 3-hydroxypropionic acid from which moisture was removed was placed in a reactor, and 63.3 mg of p-TSA as a catalyst was added at 0.2 mol% based on 3-hydroxypropionic acid. The temperature and pressure inside the reactor were maintained at 90°C and 10 torr, respectively, and the reaction was carried out for 2 hours to prepare a 3-hydroxypropionate oligomer. Then, the temperature and pressure inside the reactor were adjusted to 90°C and 0.2 torr, respectively, and the reaction was carried out for 24 hours to prepare a poly(3-hydroxypropionate).

**Comparative Example 2**

Poly(3-hydroxypropionate) was prepared in the same manner as in Comparative Example 1, except that 19.7 mg (0.2 mol%) of m-SA was used instead of 63.3 mg (0.2 mol%) of p-TSA.

**Comparative Example 3**

15 g of methyl-3-hydroxypropionate from which moisture was removed was placed in a reactor, and 19.7 mg of m-SA as a catalyst was added at 0.2 mol% based on methyl-3-hydroxypropionate. The temperature and pressure inside the reactor were maintained at 90°C and 10 torr, respectively, and the reaction was carried out for 2 hours to prepare a methyl-3-hydroxypropionate oligomer. Then, the temperature and pressure inside the reactor were adjusted to 90°C and 0.2 torr, respectively, and the reaction was carried out for 24 hours to prepare a methyl poly(3-hydroxypropionate).

**<Evaluation>**

**1. Measurement of gel permeation chromatography**

The weight average molecular weight, number average molecular weight and polydispersity index of the polymers prepared in Examples and Comparative Examples were measured by a gel permeation chromatography (GPC, Waters Alliance e2695), the results are shown in Table 2 below.

<GPC analysis conditions>

The product was dissolved in chloroform to a concentration of 1 mg/ml, then 100 $\mu\ell$ of the solution was injected into GPC, and GPC analysis was carried out at 40°C. At this time, chloroform was used as the mobile phase for GPC, the flow rate was 1.0 mL/min, and the column used was two Agilent Mixed-B units connected in series. RI Detector was used as a detector. The values of Mw were obtained using a calibration curve formed using a polystyrene standard sample. Twelve kinds of the polystyrene standard samples were used with the weight average molecular weight of 162 g/mol, 580 g/mol, 1,180 g/mol, 4,870 g/mol, 9,310 g/mol, 17,120 g/mol, 75,050 g/mol, 200,500 g/mol, 448,500 g/mol, 10,690,000 g/mol, 3,022,000 g/mol, and 6,545,000 g/mol.

**2. Evaluation of yield**

The yield of the polymers prepared in Examples and Comparative Examples was calculated according to the following Mathematical Equations 2 and 3, and the results are shown in Table 2 below.

Theoretical value = Amount of alkyl-3-hydroxypropionate added (g) * (Polymer molecular weight/Alkyl-3-hydroxypropionate molecular weight) <Mathematical Formula 2>

<Mathematical Equation 3>

Yield (%) = Yield of finally prepared polymer / Theoretical value* 100

**3. Analysis of content of vinyl group in end group**

For the polymers manufactured in the above Examples 1 to 3, 11 and Comparative Examples 1 to 3, the ratio of a vinyl group to total end group was calculated through $^1$H-NMR measurement using Buker 500MHz NMR model equipment. Specifically, each polymer was dissolved in d-$CDCl_3$ at a concentration of 8 mg/ml and measured, and calculated according to the following Mathematical Equation 1, and the results are shown in Table 2 below.

[Mathematical Equation 1]

$$vinyl\ group(\%) = \frac{2a}{(2a+b)} \times 100$$

in Mathematical Equation 1,

a is the area value of $^1$H in C=C of 6.3 ppm,
b is the area value of $^2$H in HO-$CH_2$- of 3.8 ppm.

**4. Measurement of polymer acid value**

The acid values of the polymers prepared in Examples 1 to 3, 11 and Comparative Examples 1 to 3 were measured according to the ASTM D4662 standard, and the results are shown in Table 3 below.

Specifically, the polymers were titrated with a 0.02 N potassium methoxide solution as a titration solution using a DGi 116-solvent electrode on a Mettler Toledo T5 equipment to analyze the titration point.

[Table 2]

| | Number average molecular weight | Weight average molecular weight | Polydispersity index | Yield(%) |
|---|---|---|---|---|
| Comparative Example 1 | 9,990 | 21,180 | 2.12 | 89 |
| Comparative Example 2 | 4,976 | 10,984 | 2.20 | 87 |
| Comparative Example 3 | 2,248 | 4,295 | 1.91 | 19 |
| Example 1 | 2,638 | 5,025 | 1.90 | 21 |
| Example 2 | 7,090 | 12,094 | 1.71 | 72 |
| Example 3 | 9,948 | 19,352 | 1.95 | 77 |
| Example 4 | 6,234 | 11,649 | 1.87 | 71 |
| Example 5 | 9,227 | 19,627 | 2.13 | 96.6 |
| Example 6 | 3,302 | 63,858 | 19.34 | 98 |
| Example 7 | 2,326 | 78,459 | 33.73 | 94 |
| Example 8 | 838 | 1,577 | 1.88 | 75 |
| Example 9 | 457 | 664 | 1.45 | 39 |
| Example 10 | 2,331 | 3,023 | 1.30 | 94 |
| Example 11 | 7,259 | 14,484 | 2.00 | 95 |
| Example 12 | 1,525 | 12,450 | 8.16 | 88 |
| Example 13 | 4,615 | 15,082 | 3.27 | 90 |
| Example 14 | 4,459 | 12,317 | 2.76 | 92 |
| Example 15 | 3,195 | 34,947 | 10.94 | 89 |

[Table 3]

| | Number average molecular weight | Vinyl group content (%) |
|---|---|---|
| Comparative Example 1 | 9990 | 13.8 |
| Comparative Example 2 | 4976 | 5.2 |
| Comparative Example 3 | 2248 | 0 |
| Example 1 | 2638 | 0 |
| Example 2 | 7090 | 0 |
| Example 3 | 9948 | 0 |
| Example 11 | 7259 | 0 |

[Table 4]

| | Number average molecular weight | Acid value(unit: meq/Kg) |
|---|---|---|
| Comparative Example 1 | 9990 | 198.7 |
| Comparative Example 2 | 4976 | 344.1 |
| Comparative Example 3 | 2248 | 19.4 |

(continued)

| | Number average molecular weight | Acid value(unit: meq/Kg) |
|---|---|---|
| Example 1 | 2638 | 15.2 |
| Example 2 | 7090 | 203.1 |
| Example 3 | 9948 | 128.3 |
| Example 11 | 7259 | 207.5 |

Referring to Table 2, it was confirmed that the polymers of Examples prepared from ethyl-3-hydroxypropionate, tert-butyl-3-hydroxypropionate, or n-butyl-3-hydroxypropionate are significantly higher in yield than the polymer of Comparative Example 3 prepared from methyl-3-hydroxypropionate.

In addition, referring to Table 3, it was confirmed that Examples in which polymers were prepared from alkyl-3-hydroxypropionate do not contain vinyl groups in the end groups of the polymers, unlike Comparative Examples 1 and 2 in which the polymers were prepared from 3-hydroxypropionic acid.

Furthermore, referring to Table 4, it was confirmed that the acid value of the polymer is affected by the number average molecular weight, and although Comparative Example 1 and Example 3 have similar number average molecular weights, the acid value of Example 3 is significantly lower than the acid value of Comparative Example 1. In addition, it was confirmed that although Example 1 and Comparative Example 3 have similar number average molecular weights, the acid value of Example 1 is lower than that of Comparative Example 3.

## Claims

1. A method for preparing an alkyl poly(3-hydroxypropionate) comprising:

    subjecting an alkyl-3-hydroxypropionate to condensation polymerization to prepare the alkyl poly(3-hydroxypropionate) represented by the following Chemical Formula 1,
    wherein the alkyl-3-hydroxypropionate has 2 to 20 carbon atoms in the alkyl:

    [Chemical Formula 1]

    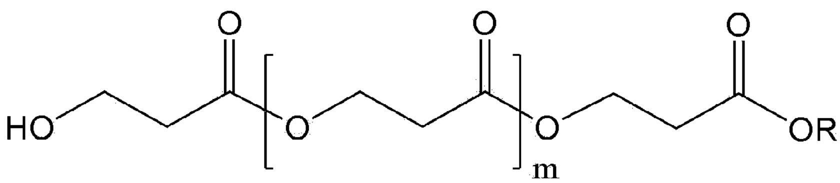

    wherein in the Chemical Formula 1,

    R is a linear or branched alkyl group represented by $C_nH_{2n+1}$ (where n is an integer of 2 to 20), and
    m is an integer of 10 or more.

2. The method for preparing an alkyl poly(3-hydroxypropionate) according to claim 1, wherein the condensation polymerization is carried out in the presence of at least one catalyst selected from the group consisting of a sulfonic acid-based catalyst, a metal oxide, a metal chloride, and a metal alkoxide catalyst.

3. The method for preparing an alkyl poly(3-hydroxypropionate) according to claim 2, wherein the catalyst is used in an amount of 0.001 mol% or more and 10 mol% or less based on the amount of the alkyl-3-hydroxypropionate.

4. The method for preparing an alkyl poly(3-hydroxypropionate) according to claim 1, wherein the condensation polymerization is carried out at a temperature of 50°C or more and 250°C or less.

5. The method for preparing an alkyl poly(3-hydroxypropionate) according to claim 1, wherein the condensation polymerization is carried out for a time period of 6 hours or more and 30 hours or less.

6. The method for preparing an alkyl poly(3-hydroxypropionate) according to claim 1, wherein the alkyl poly(3-hydroxypropionate) has a weight average molecular weight of 500 g/mol or more and 100,000 g/mol or less.

7. The method for preparing an alkyl poly(3-hydroxypropionate) according to claim 1, wherein the alkyl poly(3-hydroxypropionate) has a number average molecular weight of 300 g/mol or more and 30,000 g/mol or less.

8. The method for preparing an alkyl poly(3-hydroxypropionate) according to claim 1,

wherein the alkyl poly(3-hydroxypropionate) having a number average molecular weight of 300 g/mol or more and 3,000 g/mol or less has an acid value of 1.0 meq/Kg or more and 333.0 meq/Kg or less,
the alkyl poly(3-hydroxypropionate) having a number average molecular weight of more than 3,000 g/mol and 8,000 g/mol or less has an acid value of 300.0 meq/Kg or less, and
the alkyl poly(3-hydroxypropionate) having a number average molecular weight of more than 8,000 g/mol and 30,000 g/mol or less has an acid value of 150.0 meq/Kg or less.

9. The method for preparing an alkyl poly(3-hydroxypropionate) according to claim 1, wherein the alkyl poly(3-hydroxypropionate) has a content of vinyl groups at the end of 30 mol% or less.

10. An alkyl poly(3-hydroxypropionate) represented by the following Chemical Formula 1:

[Chemical Formula 1]

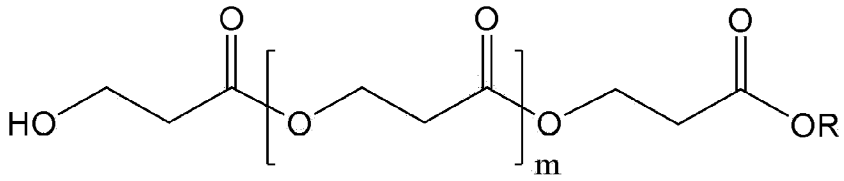

wherein in the Chemical Formula 1,

R is a linear or branched alkyl group represented by $C_nH_{2n+1}$ (where n is an integer of 2 to 20), and
m is an integer of 10 or more.

11. The alkyl poly(3-hydroxypropionate) according to claim 10,

wherein the alkyl poly(3-hydroxypropionate) having a number average molecular weight of 300 g/mol or more and 3,000 g/mol or less has an acid value of 1.0 meq/Kg or more and 333.0 meq/Kg or less,
the alkyl poly(3-hydroxypropionate) having a number average molecular weight of more than 3,000 g/mol and 8,000 g/mol or less has an acid value of 300.0 meq/Kg or less, and
the alkyl poly(3-hydroxypropionate) having a number average molecular weight of more than 8,000 g/mol and 30,000 g/mol or less has an acid value of 150.0 meq/Kg or less.

12. The alkyl poly(3-hydroxypropionate) according to claim 10, wherein the alkyl poly(3-hydroxypropionate) has a content of vinyl groups at the end of 30 mol% or less.

13. An alkyl poly(3-hydroxypropionate) composition comprising:

an alkyl poly(3-hydroxypropionate) represented by the following Chemical Formula 1, and
an alcohol having 2 to 20 carbon atoms:

[Chemical Formula 1]

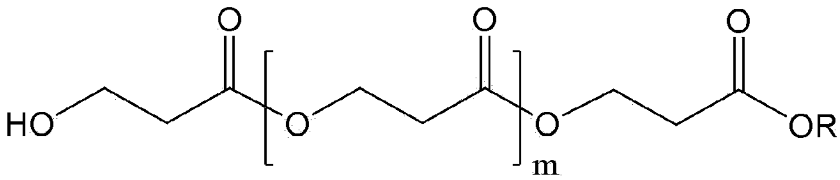

wherein in the Chemical Formula 1,

R is a linear or branched alkyl group represented by $C_nH_{2n+1}$ (where n is an integer of 2 to 20), and m is an integer of 10 or more.

**14.** The alkyl poly(3-hydroxypropionate) composition according to claim 13,

wherein the alkyl poly(3-hydroxypropionate) having a number average molecular weight of 300 g/mol or more and 3,000 g/mol or less has an acid value of 1.0 meq/Kg or more and 333.0 meq/Kg or less,
the alkyl poly(3-hydroxypropionate) having a number average molecular weight of more than 3,000 g/mol and 8,000 g/mol or less has an acid value of 300.0 meq/Kg or less, and
the alkyl poly(3-hydroxypropionate) having a number average molecular weight of more than 8,000 g/mol and 30,000 g/mol or less has an acid value of 150.0 meq/Kg or less.

**15.** The alkyl poly(3-hydroxypropionate) composition according to claim 13,
wherein the alkyl poly(3-hydroxypropionate) has a content of vinyl groups at the end of 30 mol% or less.

## INTERNATIONAL SEARCH REPORT

International application No.
**PCT/KR2023/021358**

**A. CLASSIFICATION OF SUBJECT MATTER**

**C08G 63/60**(2006.01)i; **C08G 63/82**(2006.01)i; **C08L 67/02**(2006.01)i; **C08K 5/05**(2006.01)i

According to International Patent Classification (IPC) or to both national classification and IPC

**B. FIELDS SEARCHED**

Minimum documentation searched (classification system followed by classification symbols)

C08G 63/60(2006.01); C08F 4/06(2006.01); C08G 63/06(2006.01); C08G 63/08(2006.01); C08G 63/78(2006.01); C08G 63/91(2006.01); C09J 171/10(2006.01); C09J 4/06(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 알킬-3-하이드록시프로피오네이트(alkyl-3-hydroxypropionate), 축중합(polycondensation), 알킬 폴리(3-하이드록시프로피오네이트)(alkyl poly(3-hydroxypropionate))

**C. DOCUMENTS CONSIDERED TO BE RELEVANT**

| Category* | Citation of document, with indication, where appropriate, of the relevant passages | Relevant to claim No. |
|---|---|---|
| X | JP 06-329774 A (NIPPON SHOKUBAI CO., LTD.) 29 November 1994 (1994-11-29)<br>See the claims; paragraphs [0010], [0014]-[0016], [0018] and [0019]; and example 1. | 1-15 |
| X | US 2007-0083019 A1 (ZHANG, D. et al.) 12 April 2007 (2007-04-12)<br>See claims 1, 3 and 7. | 10 |
| A | KR 10-2022-0039621 A (LG CHEM, LTD.) 29 March 2022 (2022-03-29)<br>See entire document. | 1-15 |
| A | KR 10-2021-0038251 A (LG CHEM, LTD.) 07 April 2021 (2021-04-07)<br>See entire document. | 1-15 |
| A | JP 2001-181595 A (SUMITOMO BAKELITE CO., LTD.) 03 July 2001 (2001-07-03)<br>See entire document. | 1-15 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 April 2024** | **01 April 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2023/021358**

| Patent document cited in search report | Publication date (day/month/year) | Patent family member(s) | Publication date (day/month/year) |
|---|---|---|---|
| JP 06-329774 A | 29 November 1994 | None | |
| US 2007-0083019 A1 | 12 April 2007 | US 7714097 B2 | 11 May 2010 |
| KR 10-2022-0039621 A | 29 March 2022 | None | |
| KR 10-2021-0038251 A | 07 April 2021 | None | |
| JP 2001-181595 A | 03 July 2001 | JP 3425546 B2 | 14 July 2003 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020220183172